# EUROPEAN PATENT APPLICATION

(11) **EP 3 285 193 A1**
(43) Date of publication of application: **21.02.2018**
(21) Application number: 15889288.5
(22) Date of filing: 11.06.2015
(51) Int. Cl.: G06F 19/22, G06F 19/10, C12Q 1/68

(54) **METHOD FOR PREDICTING ORGAN TRANSPLANT REJECTION USING NEXT-GENERATION SEQUENCING**

(30) Priority: 14.04.2015 KR 20150052649
(71) Applicant: Eone Diagnomics Genome Center Co., Ltd., Incheon 406-840 (KR)
(72) Inventor: LEE, Min Seob, Incheon 406-840 (KR); KWON, Sun Jae, Incheon 406-840 (KR)
(74) Representative: dompatent von Kreisler Selting Werner - Partnerschaft von Patent- und Rechtsanwälten mbB
(86) International application number: PCT/KR2015/005905
(87) International publication number: WO 2016/167408

(57) **Abstract**

The present invention relates to a method for non-invasively predicting an organ transplant rejection by measuring the ratio between an organ-donor-specific nucleic acid sequence and a recipient-specific nucleic acid sequence in a biological sample of an organ transplant recipient and, more specifically, to a method for predicting an organ transplant rejection by measuring the ratio of a donor-derived marker sequence and a recipient-derived marker sequence regarding three or more markers selected in Tables 1 to 10 by testing a biological sample (e.g. blood) of an organ transplant recipient and predicting the organ transplant rejection based on the measurement. The method for predicting an organ transplant rejection using next-generation sequencing (NGS) according to the present invention or a digital base amplification technique can apply even to a very small amount of sample, is faster and inexpensive, is fast in the rate of data analysis, can apply to all organ transplants of all races in the word, and can simultaneously detect error probability of a sequence analysis, and thus is useful to non-invasively predict the organ transplant rejection.

## Description

### TECHNICAL FIELD

The present invention relates to a method of non-invasively predicting organ transplant rejection by measuring the ratio between donor-specific nucleic acid sequences and recipient-specific nucleic acid sequences in a biological sample obtained from an organ transplant recipient, and more particularly to a method of predicting organ transplant rejection based on the results of measuring the ratio between donor-derived marker sequences and recipient-derived marker sequences by analyzing a biological sample (e.g., blood) obtained from an organ transplant recipient.

### BACKGROUND ART

Accurate and timely diagnosis of organ transplant rejection in an organ transplant recipient is essential for survival of the organ transplant recipient. However, methods for diagnosing organ transplant rejection, which are currently used, have many disadvantages. For example, gold standard for diagnosing heart transplant rejection is examining tissue at each time point with surgery for heart biopsy, however, this methods shows many problems including high costs, variability between tissue biopsy physicians, and severe patient discomfort (F. Saraiva et al., Transplant. Proc. Vol. 43, pp. 1098-1012, 2011).

In order to overcome such limitations, non-invasive methods have been used, such as a method for measuring gene expression signals which tend to increase when organ transplant rejection occurs, a method for measuring the level of immune proteins, and the like. However, these methods also pose limitations as they tend to produce high false positive results due to the complex cross-reactivity of various immune responses, and are based on tissue-specific gene expression signals.

In the late 1990s, cell-free donor-derived DNA (cfdDNA) was detected in the urine and blood of organ transplant recipients (J. Zhang et al., Clin. CHem. Vol. 45, pp. 1741-1746, 1999., Y. M. Lo et al., Lancet, Vol. 351, pp. 1329-1330, 1998). Based on this finding, methods for non-invasive diagnosis of organ transplant rejection have been proposed. For example, donor-specific DNA in a female recipient of organ from a male donor can be analyzed using various molecular and chemical assays that detect Y chromosome (T. K. Sigdel et al., Transplantation, Vol. 96, pp. 97-101, 2013). However, the cfdDNA is present in minute quantity, whereas the background DNA is present in abundance. Thus, a highly specific and sensitive method for analyzing this cfdDNA is required.

Next-generation sequencing (NGS) has the capacity of overcoming such limitations and is becoming more and more popular. The next-generation sequencing technique can produce huge amount of data within a short span of time, unlike the existing methods. Thus, this technique is both time and cost effective for individual genome sequencing. The next-generation sequencing technique also provides an unprecedented opportunity to detect disease-causing genes in Mendelian diseases, rare diseases, cancers and the like. Extraordinary progress has been made on genome sequencing platforms and the sequencing data analysis costs have gradually reduced. In the next-generation sequencing technique, DNA is extracted from a sample and mechanically fragmented, followed by size-specific library construction which is used for sequencing. Initial sequencing data are produced while repeating the association and dissociation of four complementary nucleotides with one base unit by using high-throughput sequencing system. Subsequently, bioinformatics-based analysis steps are performed which includes initial data trimming, mapping, genetic mutation identification, and mutation annotation. The analysis leads to the identification of genetic mutations that may affect diseases and various biological phenotypes. Thus, the next-generation sequencing technique contributes to the creation of new added values through the development and commercialization of new therapeutic agents. The next-generation sequencing technique can not only be used for DNA analysis, but also for RNA and methylation analysis. This includes whole-exome sequencing (WES) that captures and sequences only protein-encoding exome regions. This whole-exome sequencing technique is a method that produce sequences of the region encoding a protein having the most direct connection with the development of disease. This technique is widely used, because sequencing of only the exome region is more cost-effective as compared to sequencing of the whole genome. The modification of the whole-exome sequencing technique is popularly known as targeted sequencing. This sequencing technique has the capacity of detecting genetic mutation in the region of interest by using a designed probe. The probe captures only the genetic region of interest, which in turn is used for the detection of genetic mutation in the major oncogene of interest. This technique is relatively easy to perform and can be achieved by significantly lower costs. This sequencing technique is referred to as targeted sequencing.

It is a well-known fact that the use of this next-generation sequencing technique makes it possible to analyze all nucleic acids present in a sample, and thus is highly useful for the analysis of cfdDNA that is present in a desired sample at a very low concentration. For example, Iwijin De Vlaminck et al. performed the analysis of 565 samples obtained from 65 heart transplant patients over time which indicated that the level of cfdDNA in the samples from the recipients were elevated when organ transplant rejection appeared (Iwijin De Vlaminck et al., Sci. Transl. Med. Vol. 6, 241ra77, 2014).

However, this method has limitations; it requires considerable amount of time and cost as it analyzes whole genome data.

Accordingly, the present inventors have made extensive efforts to solve the above-described problems, and as a result, have found that, when markers shown in Table 1 to 10 below are amplified to a size of less than 200 bp and used in next-generation sequencing, cfDNA in a sample can be used intact and, at the same time, analysis sensitivity and accuracy are maintained and analysis time and cost are significantly decreased, thereby completing the present invention.

### DISCLOSURE OF INVENTION

### TECHNICAL PROBLEM

It is an object of the present invention to provide a method of predicting organ transplant rejection in a biological sample, obtained from a recipient who received an organ from a donor, by next-generation sequencing (NGS) or digital base amplification

Another object of the present invention is to provide a computer system comprising a computer readable medium encoded with a plurality of instructions for controlling a computing system to perform an operation of predicting organ transplant rejection in a biological sample, obtained from a recipient who received an organ from a donor, by use of next-generation sequencing (NGS) or digital base amplification.

### TECHNICAL SOLUTION

To achieve the above object, the present invention provides a method of predicting organ transplant rejection in a biological sample, obtained from a recipient who received an organ from a donor, by next-generation sequencing (NGS) or digital base amplification, the method comprising the steps of:
non-invasively obtaining a biological sample, which contains donor-derived and recipient-derived cell-free nucleic acid molecules, from a recipient who received an organ from a donor;
amplifying three or more marker sequences, selected from marker sequences shown in Tables 1 to 10, in cell-free nucleic acid molecules isolated from the biological sample;
analyzing the amplified sequences by next-generation sequencing (NGS) or digital base amplification;
based on the analysis of the sequences, determining the ratio between each of the donor-derived marker sequences and each of the recipient-derived marker sequences; and
comparing the ratio with one or more cutoff values.

The present invention also provides a method of predicting organ transplant rejection in a biological sample, obtained from a recipient who received an organ from a donor, by next-generation sequencing (NGS) or digital base amplification, the method comprising the steps of:
non-invasively obtaining a biological sample, which contains donor-derived and recipient-derived cell-free nucleic acid molecules, from a recipient who received an organ from a donor;
amplifying three or more marker sequences, selected from marker sequences shown in Tables 1 to 10, in cell-free nucleic acid molecules isolated from the biological sample;
analyzing the amplified sequences by next-generation sequencing (NGS) or digital base amplification;
based on the analysis of the sequences, determining the ratio between each of the donor-derived marker sequences and each of the recipient-derived marker sequences; and
measuring the ratio over time, and predicting whether the recipient will have transplant rejection, graft dysfunction or organ failure when the ratio each of the donor-derived marker sequences increases.

The present invention also provides a computer system comprising a computer readable medium encoded with a plurality of instructions for controlling a computing system to perform an operation of predicting organ transplant rejection in a biological sample, obtained from a recipient who received an organ from a donor, by use of next-generation sequencing (NGS) or digital base amplification,
wherein the biological sample contains donor-derived and recipient-derived cell-free nucleic acid molecules from a recipient who received an organ from a donor, and
wherein the operation comprises the steps of:
receiving data obtained by analyzing three or more marker sequences, selected from markers shown in Tables 1 to 10, in the cell-free nucleic acid molecules isolated from the biological sample, by use of next-generation sequencing (NGS) or digital base amplification;
based on the analysis of the sequences, determining the ratio between each of the donor-derived marker sequences and each of the recipient-derived marker sequences;
comparing the ratio with one or more cutoff values; and
based on the comparison, predicting whether or not organ transplant rejection in the recipient will be present.

### BRIEF DESCRIPTION OF THE DRAWINGS

FIG. 1 is a conceptual view depicting a method for the prediction of organ transplant rejection by next-generation sequencing (NGS). As shown in FIG. 1, a biological sample (e.g., blood) is collected non-invasively from a patient, and circulating cell-free DNA is isolated from the biological sample. A selected marker set in the sample is amplified by multiplexed PCR and analyzed by short read length next generation sequencing to count the ratio between marker alleles, thereby predicting organ transplant rejection.
FIG. 2 illustrates that when a single marker is amplified using a designed primer and analyzed, NGS can be very quickly performed because a target SNP site is located immediately following the primer.
FIG. 3 depicts the results obtained by mixing DNAs to artificially make organ transplantation conditions for 2023 markers selected from markers shown in Table 1 to 10, and measuring the percentages of donor-derived SNP markers in a transplant recipient.
FIG. 4 depicts the results of measuring each SNP marker in a sample comprising artificially mixed DNAs.

### ADVANTAGEOUS EFFECTS

The method of prediction of organ transplant rejection by next-generation sequencing (NGS) or digital base amplification according to the present invention is applicable even for minute amount of sample. This method is rapid, inexpensive, enables rapid data analysis, and is applicable irrespective of the types of organs and races in the world, Also it can detect the probability of the sequencing error. Thus, the method of the present invention is vital for non-invasive prediction of organ transplant rejection.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods, which will be described below, are those well-known and commonly employed in the art.

As used herein, the term "next-generation sequencing (NGS)" means a technique in which the whole genome is fragmented and the fragments are sequenced in a high-throughput manner. The term includes the technologies of Agilent, Illumina, Roche and Life Technologies. In a broad sense, the term includes third-generation sequencing technologies such as Pacificbio, Nanopore Technology and the like, and also the fourth-generation sequencing technologies.

As used herein, the term "organ transplant rejection" includes both acute and chronic transplant rejections. "Acute transplant rejection (AR)" occurs when the donor's organ is considered exogenous by the recipient's immune system. "Acute transplant rejection" implies that the recipient's immune cells penetrate a transplanted organ, resulting in destruction of the transplant organ. Acute transplant rejection occurs very rapidly, and it generally occurs within weeks after organ transplantation surgery. Generally, acute transplant rejection can be inhibited or suppressed by immunosuppressants such as rampamycin, cyclosprin A, anti-CD4 monoclonal antibody and the like. "Chronic transplant rejection (CR)" generally occurs within several months or years after organ transplantation. Organ fibrosis that occurs in all kinds of chronic transplant rejection is a common phenomenon that reduces the function of each organ. For example, chronic transplant rejection in a transplanted lung occurs leads to fibrotic reaction which destroys the airways leading to pneumonia (bronchiolitis obliterans). Furthermore, when chronic transplant rejection occurs in a transplanted heart, it will result in fibrotic atherosclerosis. Similarly, the chronic transplant rejection in a transplanted kidney leads to obstructive nephropathy, nephrosclerosis, tubulointerstitial nephropathy or the like. Chronic transplant rejection also results in ischemic insult, denervation of a transplanted organ,hyperlipidemia and hypertension symptoms associated with immunosuppressants. As used herein, the term "biological sample" refers to any sample that is obtained from a recipient and contains one or more nucleic acid molecule(s) of interest.

The term "nucleic acid" or "polynucleotide" refers to a deoxyribonucleic acid (DNA) or ribonucleic acid (RNA) and a polymer thereof in either single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res.19:5081 (1991); Ohtsuka et al., J. Biol. Chem. 260:2605-2608 (1985); and Rossolini et al, MoI. Cell. Probes 8:91-98 (1994)). The term nucleic acid is used interchangeably with gene, cDNA, mRNA, small noncoding RNA, micro RNA (miRNA), Piwi-interacting RNA, and short hairpin RNA (shRNA) encoded by a gene or locus.

As used herein, the term "single nucleotide polymorphism (SNP)" refers to a single nucleotide difference between a plurality of individuals within a single species. For example, when an rs7988514 marker present in chromosome 13 of a transplant donor is C/G and the marker in a transplant recipient is T/A, the method of the present invention can be used to analyze the ratio of the donor-derived marker in the blood of the recipient to thereby predict organ transplant rejection.

The term "cutoff value" as used herein means a numerical value whose value is used to arbitrate between two or more states (e.g. normal state and organ transplant rejection state) of classification for a biological sample. For example, if the ratio of a donor-derived marker in the blood of a recipient is greater than the cutoff value, the recipient is classified as being in the organ transplant rejection state; or if the ratio of the donor-derived marker in the blood of the recipient is less than the cutoff value, the recipient is classified as being in the normal state.

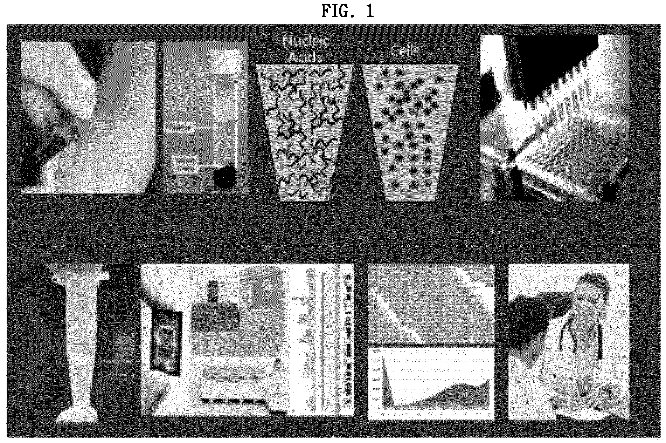

In the present invention, it was found that when a biological sample collected from a recipient is analyzed by short read length next-generation sequencing or digital base amplification by using at least three markers selected from the list of markers shown in Tables 1 to 10 (FIG. 1), organ transplant rejection in the biological sample can be quickly predicted with high accuracy.

Therefore, in one aspect, the present invention is directed to a method of predicting organ transplant rejection in a biological sample, obtained from a recipient who received an organ from a donor, by next-generation sequencing (NGS) or digital base amplification, the method comprising the steps of:
non-invasively obtaining a biological sample, which contains donor-derived and recipient-derived cell-free nucleic acid molecules, from a recipient who received an organ from a donor;
amplifying three or more marker sequences, selected from marker sequences shown in Tables 1 to 10, in cell-free nucleic acid molecules isolated from the biological sample;
analyzing the amplified sequences by next-generation sequencing (NGS) or digital base amplification;
based on the analysis of the sequences, determining the ratio between each of the donor-derived marker sequences and each of the recipient-derived marker sequences; and
comparing the ratio with one or more cutoff values.

In the present invention, the marker sequences listed in Tables 1 to 10 can be used as single nucleotide polymorphism (SNP) markers which are bi-allelic, are in agreement with the a Hardy-Weinberg distribution and have a minor allele frequency of 0.4 or greater.

In the present invention, the marker numbers (rs numbers) listed in Tables 1 to 10 may have reference SNP numbers that can be searched in dbSNP database (http://www.ncbi.nlm.nih.gov/snp) of NCBI.

**Table 1: Markers used in the present invention**

| Marker number | Marker number | Marker number | Marker number | Marker number | Marker number | Marker number |
|---|---|---|---|---|---|---|
| rs1000160 | rs1483303 | rs2296122 | rs3773445 | rs6565831 | rs899968 | rs9978408 |
| rs1000501 | rs1491658 | rs2296348 | rs377685 | rs6565969 | rs904654 | rs9979609 |
| rs1002460 | rs1495816 | rs2297256 | rs378108 | rs6565990 | rs906629 | rs9980589 |
| rs1003092 | rs1495965 | rs2297291 | rs3786203 | rs6566067 | rs912441 | rs9980734 |
| rs10035179 | rs1498553 | rs2298437 | rs3786355 | rs6566186 | rs912697 | rs9980852 |
| rs10048391 | rs1501230 | rs2298583 | rs3787732 | rs6566286 | rs914163 | rs9980934 |
| rs10048862 | rs1501233 | rs2318993 | rs3788190 | rs6566554 | rs914231 | rs9981016 |
| rs10049125 | rs1501871 | rs2320747 | rs3788200 | rs6566669 | rs914232 | rs9982310 |
| rs10057967 | rs1506008 | rs232374 | rs378872 | rs6566675 | rs914532 | rs9982473 |
| rs1006757 | rs1508494 | rs232381 | rs3795494 | rs6566862 | rs915800 | rs9983057 |
| rs10069510 | rs1511151 | rs2328975 | rs379605 | rs6567221 | rs915876 | rs9983351 |
| rs1007300 | rs1512473 | rs2329327 | rs3802981 | rs6579927 | rs918823 | rs9983568 |
| rs10074004 | rs1518036 | rs2330396 | rs3803196 | rs659897 | rs924895 | rs9984531 |
| rs10075717 | rs1519126 | rs2330572 | rs3805015 | rs660207 | rs926130 | rs9985011 |
| rs10078065 | rs1526589 | rs2332023 | rs3806 | rs660236 | rs928299 | rs9985019 |
| rs10083274 | rs1530330 | rs2332026 | rs3809346 | rs660622 | rs9285110 | rs9985057 |
| rs10085762 | rs153119 | rs2332240 | rs3810590 | rs660811 | rs9285254 | rs999104 |
| rs1009823 | rs153283 | rs233616 | rs3817 | rs661100 | rs9285297 | |
| rs10098835 | rs1532846 | rs233621 | rs3819177 | rs661293 | rs9292170 | |
| rs1010392 | rs1533434 | rs2337483 | rs3826616 | rs662792 | rs9300377 | |
| rs1010559 | rs1535904 | rs234787 | rs3844038 | rs6650458 | rs9300518 | |
| rs1013059 | rs1536780 | rs235310 | rs384901 | rs6650723 | rs9300569 | |
| rs10140137 | rs1536807 | rs235329 | rs3850193 | rs665479 | rs9300647 | |
| rs1014209 | rs1542578 | rs236043 | rs3853682 | rs6662560 | rs9300921 | |
| rs1014604 | rs1545310 | rs2362839 | rs385501 | rs6678950 | rs9301149 | |
| rs1015820 | rs1549060 | rs2366188 | rs3856791 | rs6680365 | rs9301441 | |
| rs10158288 | rs1553108 | rs2388919 | rs3864997 | rs671441 | rs9301695 | |
| rs10164030 | rs1553295 | rs2390878 | rs3865418 | rs673220 | rs930189 | |
| rs1018676 | rs1554936 | rs2390998 | rs3865419 | rs674929 | rs9303869 | |
| rs10210 | rs1556817 | rs239340 | rs3866900 | rs6762432 | rs9303900 | |
| rs10222177 | rs1560669 | rs2395891 | rs386838 | rs6769917 | rs9304336 | |

**Table 2: Markers used in the present invention**

| Marker number | Marker number | Marker number | Marker number | Marker number | Marker number | Marker number |
|---|---|---|---|---|---|---|
| rs1058396 | rs1682914 | rs2571764 | rs4268850 | rs703505 | rs946228 | rs10915584 |
| rs10742709 | rs1690551 | rs2575177 | rs428424 | rs7062 | rs946231 | rs10935501 |
| rs10744938 | rs169332 | rs2576036 | rs4284535 | rs706466 | rs949741 | rs1757889 |
| rs1075906 | rs16943649 | rs2576038 | rs4284740 | rs7067226 | rs949931 | rs17591231 |
| rs1077550 | rs16950864 | rs2581732 | rs429133 | rs7067297 | rs950408 | rs277665 |
| rs10780042 | rs16951141 | rs2585481 | rs430043 | rs7099777 | rs9506275 | rs2779134 |
| rs10781417 | rs16951664 | rs2585495 | rs4306614 | rs7139787 | rs9506919 | rs445593 |
| rs10790400 | rs16978368 | rs2586776 | rs431864 | rs7139997 | rs9507577 | rs4456612 |
| rs1079139 | rs16980558 | rs2586778 | rs4319623 | rs7140005 | rs9507631 | rs7234111 |
| rs1079174 | rs16980586 | rs2586779 | rs432294 | rs714831 | rs950772 | rs7234383 |
| rs10799636 | rs16980588 | rs2587428 | rs4329028 | rs716117 | rs9508080 | rs9521192 |
| rs10840837 | rs17041964 | rs25876 | rs4346468 | rs716510 | rs9508327 | rs952134 |
| rs10851201 | rs17069898 | rs2591518 | rs4346469 | rs7186326 | rs9508716 | rs10903035 |
| rs10853392 | rs17070149 | rs2628125 | rs4349043 | rs7206898 | rs9509249 | rs10915311 |
| rs10853603 | rs17071467 | rs2641114 | rs4349054 | rs721247 | rs9509441 | rs1754514 |
| rs10854400 | rs17080696 | rs2641962 | rs435081 | rs7226953 | rs9509516 | rs17550441 |
| rs10856953 | rs17084208 | rs2687899 | rs4372773 | rs7226979 | rs9510334 | rs2776341 |
| rs10858469 | rs170962 | rs269286 | rs4375553 | rs7227268 | rs9510340 | rs2776344 |
| rs10866988 | rs17184424 | rs2699323 | rs4380323 | rs7228099 | rs9510597 | rs4452046 |
| rs10869149 | rs1720839 | rs271374 | rs438064 | rs7228812 | rs9510775 | rs4454841 |
| rs10869157 | rs17232531 | rs271397 | rs4383238 | rs7229278 | rs9512046 | rs7233802 |
| rs10870724 | rs17248234 | rs2729429 | rs4384683 | rs7229644 | rs9512063 | rs7233985 |
| rs10870932 | rs1725235 | rs2736084 | rs4389803 | rs7229967 | rs9514560 | rs9520400 |
| rs10871180 | rs17307670 | rs273696 | rs439146 | rs722998 | rs9514663 | rs9521146 |
| rs10871550 | rs17326281 | rs273701 | rs4398676 | rs7230288 | rs9515124 | |
| rs10871620 | rs1734848 | rs2747740 | rs4402665 | rs7230661 | rs9515621 | |
| rs10871641 | rs17351137 | rs275948 | rs4402842 | rs7230860 | rs9515774 | |
| rs10871815 | rs17363863 | rs2762171 | rs4407150 | rs7231029 | rs9516644 | |
| rs10875612 | rs174047 | rs2764618 | rs4409964 | rs7231046 | rs9516904 | |
| rs10880836 | rs1748124 | rs2765327 | rs4428160 | rs7231112 | rs9518743 | |
| rs10889256 | rs17513940 | rs2774494 | rs4430618 | rs7231366 | rs9518903 | |
| rs10889523 | rs17518254 | rs2775138 | rs4440160 | rs7232672 | rs9518972 | |
| rs10899035 | rs17533 | rs2775537 | rs444435 | rs7233515 | rs9520132 | |

**Table 3: Markers used in the present invention**

| Marker number | Marker number | Marker number | Marker number | Marker number | Marker number | Marker number |
|---|---|---|---|---|---|---|
| rs10937406 | rs17591266 | rs2780746 | rs446716 | rs7234990 | rs9521472 | rs11151454 |
| rs10937408 | rs17686332 | rs2782462 | rs4468699 | rs7235005 | rs9521488 | rs11151514 |
| rs10942130 | rs17711702 | rs2783084 | rs448247 | rs7235160 | rs9521801 | rs1790428 |
| rs10955093 | rs1772587 | rs2786712 | rs448503 | rs7235654 | rs9521832 | rs1790584 |
| rs10955174 | rs17740268 | rs2793734 | rs4495668 | rs7235891 | rs9521853 | rs2825610 |
| rs10955420 | rs1774918 | rs2793736 | rs4497518 | rs7235930 | rs9522262 | rs2825688 |
| rs10972552 | rs17754863 | rs2794243 | rs4508511 | rs7235989 | rs9523648 | rs466448 |
| rs1102617 | rs17765723 | rs2794247 | rs4510132 | rs7236090 | rs9524400 | rs467140 |
| rs1105513 | rs17781378 | rs2803220 | rs451826 | rs7236427 | rs9525095 | rs7277441 |
| rs1105856 | rs1778797 | rs2803348 | rs4520729 | rs7236653 | rs9525149 | rs7277926 |
| rs11069237 | rs17794801 | rs2807441 | rs4522508 | rs7237517 | rs9525158 | rs9532436 |
| rs11071215 | rs1779843 | rs2821796 | rs4525375 | rs7237577 | rs9525300 | rs9533146 |
| rs11080646 | rs17800754 | rs2822618 | rs4539677 | rs7237747 | rs9525641 | rs11151426 |
| rs11081004 | rs17804894 | rs2822648 | rs4544336 | rs7237774 | rs9525643 | rs11151452 |
| rs11081037 | rs1783099 | rs2822661 | rs455508 | rs7239234 | rs9526222 | rs1788648 |
| rs11081555 | rs1783305 | rs2822809 | rs455921 | rs723940 | rs9526312 | rs1788658 |
| rs11082705 | rs1783395 | rs2822965 | rs4573787 | rs7240004 | rs9526400 | rs2825583 |
| rs11083008 | rs1783404 | rs2822973 | rs4576968 | rs7240257 | rs9526792 | rs2825608 |
| rs11083386 | rs17836226 | rs2822975 | rs458029 | rs7240294 | rs9527084 | rs4661514 |
| rs1108522 | rs1785739 | rs2823145 | rs4583369 | rs7240363 | rs9527138 | rs466277 |
| rs11088040 | rs1785745 | rs2823152 | rs4588087 | rs7240404 | rs9527905 | rs7276176 |
| rs11088302 | rs1786388 | rs2823169 | rs4588273 | rs7240429 | rs9528696 | rs7277076 |
| rs11088405 | rs1786427 | rs2823795 | rs4611350 | rs7241051 | rs9528931 | rs9531615 |
| rs11088861 | rs1786648 | rs2823809 | rs4613170 | rs7241461 | rs9529287 | rs9532420 |
| rs11096435 | rs1787013 | rs2823983 | rs4617713 | rs7241510 | rs9529809 | |
| rs11096453 | rs1787186 | rs2824133 | rs461853 | rs7241718 | rs9529814 | |
| rs1111937 | rs1787292 | rs2824238 | rs4628 | rs7242966 | rs9530505 | |
| rs11135235 | rs1787301 | rs2824376 | rs4638449 | rs7243620 | rs9530604 | |
| rs1114342 | rs1787337 | rs2824762 | rs4650520 | rs7244347 | rs9530721 | |
| rs11148802 | rs1787435 | rs2825516 | rs4653036 | rs7245332 | rs9530981 | |
| rs11150946 | rs1787557 | rs2825560 | rs465353 | rs725040 | rs953109 | |
| rs11151009 | rs1787577 | rs2825576 | rs465446 | rs7260507 | rs9531243 | |
| rs11151180 | rs1788002 | rs2825578 | rs4661295 | rs7275842 | rs9531587 | |

**Table 4: Markers used in the present invention**

| Marker number | Marker number | Marker number | Marker number | Marker number | Marker number | Marker number |
|---|---|---|---|---|---|---|
| rs11151657 | rs1790649 | rs2825824 | rs4677496 | rs7278004 | rs9533177 | rs11662474 |
| rs11151684 | rs1790875 | rs2826117 | rs4685212 | rs7278137 | rs9533397 | rs11662612 |
| rs11151698 | rs1792668 | rs2826259 | rs4686407 | rs7278676 | rs9533738 | rs1890306 |
| rs11151892 | rs1792674 | rs2826390 | rs4687889 | rs7279020 | rs9534174 | rs1891132 |
| rs11152060 | rs1792687 | rs2826392 | rs468837 | rs7279626 | rs9534262 | rs2828798 |
| rs11152170 | rs1806487 | rs2826395 | rs468849 | rs7280367 | rs9534330 | rs2828800 |
| rs11152242 | rs1807783 | rs2826396 | rs469303 | rs7280538 | rs9534515 | rs4799055 |
| rs11152264 | rs1808693 | rs2826399 | rs469353 | rs7280591 | rs9534596 | rs4799198 |
| rs11164166 | rs1810129 | rs2826506 | rs470490 | rs7280941 | rs9534638 | rs732569 |
| rs11167694 | rs1817141 | rs2826718 | rs4747351 | rs7281206 | rs9535880 | rs7326426 |
| rs11208377 | rs1819894 | rs2826721 | rs4750494 | rs7281674 | rs9536346 | rs9545554 |
| rs1125807 | rs1826318 | rs2826737 | rs4757240 | rs728174 | rs9536415 | rs9545559 |
| rs1143914 | rs1829651 | rs2826803 | rs4761518 | rs7281853 | rs9538268 | rs11661072 |
| rs1145560 | rs1830926 | rs2826807 | rs4770032 | rs7282582 | rs9538278 | rs11661849 |
| rs1146888 | rs1832265 | rs2826949 | rs4770463 | rs7282876 | rs9539175 | rs1888469 |
| rs1152991 | rs1833277 | rs2826959 | rs4770597 | rs7283077 | rs9539877 | rs1888514 |
| rs1153294 | rs1833304 | rs2827038 | rs4770601 | rs7283399 | rs9539893 | rs2828506 |
| rs1153295 | rs1833486 | rs2827433 | rs4770771 | rs729809 | rs9540071 | rs2828793 |
| rs1156026 | rs1834545 | rs2827527 | rs4771157 | rs7304820 | rs9540450 | rs4798412 |
| rs115750 | rs1834547 | rs2827528 | rs4771638 | rs7310809 | rs9540451 | rs4798479 |
| rs115957 62 | rs1851043 | rs2827530 | rs4771695 | rs7317338 | rs9540627 | rs7325068 |
| rs11617291 | rs1854100 | rs2827874 | rs4771833 | rs7317341 | rs9540642 | rs7325529 |
| rs11617562 | rs1855259 | rs2827965 | rs4771904 | rs7317430 | rs9541479 | rs9545224 |
| rs11617 606 | rs1864469 | rs2827987 | rs4772278 | rs7319926 | rs9541813 | rs9545244 |
| rs11618168 | rs1866337 | rs2828001 | rs4772857 | rs7319976 | rs9542105 | |
| rs11619265 | rs1866986 | rs2828023 | rs4772937 | rs7320145 | rs9542137 | |
| rs11619462 | rs1870592 | rs2828055 | rs4773212 | rs7321115 | rs9542383 | |
| rs11620473 | rs1874864 | rs2828061 | rs4773395 | rs7321584 | rs9542852 | |
| rs11659206 | rs1874921 | rs2828089 | rs4773402 | rs7322458 | rs9542951 | |
| rs11659463 | rs1876583 | rs2828151 | rs4773838 | rs7322868 | rs9542969 | |
| rs11659969 | rs188446 | rs2828155 | rs4784207 | rs7323182 | rs9543171 | |
| rs11660213 | rs1886969 | rs2828263 | rs4784376 | rs7323558 | rs9544749 | |
| rs11660737 | rs1887718 | rs2828500 | rs4796869 | rs7324970 | rs9544845 | |

**Table 5: Markers used in the present invention**

| Marker number | Marker number | Marker number | Marker number | Marker number | Marker number | Marker number |
|---|---|---|---|---|---|---|
| rs11664190 | rs1891948 | rs2828802 | rs4799910 | rs7326820 | rs9545852 | rs12184876 |
| rs11664478 | rs189204 | rs2829066 | rs4800786 | rs7326944 | rs9545853 | rs12185460 |
| rs11664727 | rs1892681 | rs2829115 | rs4800967 | rs7327180 | rs9545861 | rs1970678 |
| rs11665106 | rs1893455 | rs2829214 | rs4800970 | rs7327256 | rs9545903 | rs1972415 |
| rs11665385 | rs1893654 | rs2829432 | rs4800973 | rs7327729 | rs9546633 | rs2833935 |
| rs11701849 | rs1893657 | rs2829445 | rs4816597 | rs7329520 | rs9546677 | rs2834208 |
| rs11701901 | rs1893673 | rs2829614 | rs4816610 | rs7330025 | rs9547087 | rs4886217 |
| rs11702340 | rs1895076 | rs2829674 | rs4816681 | rs7331003 | rs9547646 | rs4890312 |
| rs1176270 | rs1898165 | rs2829887 | rs4817097 | rs7331794 | rs9548869 | rs9561936 |
| rs1183856 | rs1904177 | rs2830048 | rs4817371 | rs7332180 | rs9548880 | rs9561953 |
| rs11839815 | rs1908593 | rs2830194 | rs4817609 | rs7333280 | rs9548930 | rs1217618 |
| rs11872146 | rs1910660 | rs2830424 | rs4817685 | rs7333503 | rs9549172 | rs1218307 |
| rs11872403 | rs191482 | rs2830437 | rs4817890 | rs7333648 | rs9549293 | rs195700 |
| rs11872509 | rs1920083 | rs2830604 | rs4817891 | rs733398 | rs9551135 | rs1970668 |
| rs11872828 | rs1923732 | rs2830643 | rs4818015 | rs7334111 | rs9551233 | rs2833846 |
| rs11873161 | rs1923771 | rs2830811 | rs4818108 | rs7334546 | rs9551406 | rs2833916 |
| rs11876001 | rs1923886 | rs2830841 | rs4818144 | rs7334805 | rs9552733 | rs4885878 |
| rs11876772 | rs1924417 | rs2830856 | rs4818160 | rs7335163 | rs9552874 | rs4885880 |
| rs11877050 | rs1925857 | rs2831057 | rs4818179 | rs7335426 | rs9553022 | rs735862 |
| rs11877617 | rs1926264 | rs2831350 | rs4818561 | rs7335836 | rs9553390 | rs736081 |
| rs11910048 | rs1926614 | rs2831378 | rs4819090 | rs7335944 | rs9554579 | rs9561532 |
| rs11910807 | rs1926616 | rs2831699 | rs4819128 | rs7336089 | rs9554641 | rs9561935 |
| rs11910832 | rs1927014 | rs2831702 | rs4819130 | rs733610 | rs9555119 | |
| rs11919425 | rs1927807 | rs2831706 | rs4819201 | rs7336348 | rs9555266 | |
| rs11939712 | rs1927830 | rs2831755 | rs4835587 | rs7336658 | rs9555581 | |
| rs11948061 | rs1930586 | rs2832155 | rs483712 | rs7337326 | rs9555714 | |
| rs11959584 | rs1932917 | rs2832236 | rs4841972 | rs7337382 | rs9556425 | |
| rs11960564 | rs1933187 | rs2832916 | rs4845953 | rs7337528 | rs9560166 | |
| rs12018498 | rs1937443 | rs2833117 | rs486285 | rs7337915 | rs9560339 | |
| rs12020398 | rs1942399 | rs2833123 | rs487812 | rs7338544 | rs9560797 | |
| rs12037545 | rs1942531 | rs2833153 | rs4884402 | rs7339162 | rs9560800 | |
| rs12136961 | rs1942803 | rs2833523 | rs4884905 | rs7339250 | rs9560807 | |
| rs12149 | rs1949593 | rs2833636 | rs4884906 | rs734747 | rs9561254 | |

**Table 6: Markers used in the present invention**

| Marker number | Marker number | Marker number | Marker number | Marker number | Marker number | Marker number |
|---|---|---|---|---|---|---|
| rs12185828 | rs1972598 | rs2834295 | rs4890333 | rs742276 | rs9562045 | rs12584118 |
| rs12287199 | rs1972917 | rs2834297 | rs4890698 | rs743446 | rs9562457 | rs12584427 |
| rs1231048 | rs1979613 | rs2834337 | rs4890876 | rs7441242 | rs9562501 | rs2027667 |
| rs12326252 | rs1980080 | rs2834339 | rs4891097 | rs747781 | rs9562637 | rs2031446 |
| rs12327010 | rs1980942 | rs2834694 | rs4891098 | rs748607 | rs9563028 | rs2836404 |
| rs1236411 | rs1980950 | rs2834709 | rs4891160 | rs7504436 | rs9563770 | rs2836488 |
| rs12420519 | rs1981084 | rs2834712 | rs4891325 | rs7504842 | rs9564167 | rs4941643 |
| rs12427782 | rs1981390 | rs2834756 | rs4891576 | rs7509953 | rs9564355 | rs4941715 |
| rs12428610 | rs1982837 | rs2834782 | rs4891734 | rs7544781 | rs9564535 | rs7735484 |
| rs12428798 | rs1986899 | rs2834796 | rs4907464 | rs754777 | rs9564577 | rs7799930 |
| rs12454023 | rs1988657 | rs2834884 | rs4907552 | rs756040 | rs9564626 | rs9574824 |
| rs12454180 | rs1991753 | rs2834908 | rs4910359 | rs760345 | rs9564747 | rs9574897 |
| rs12454706 | rs1993355 | rs2835035 | rs4911045 | rs7614 | rs9564791 | rs12583161 |
| rs12455429 | rs199667 | rs2835043 | rs4920104 | rs7616178 | rs9565398 | rs12583202 |
| rs12456484 | rs1997353 | rs2835103 | rs4920520 | rs7618973 | rs9565654 | rs2026744 |
| rs12457067 | rs1998956 | rs2835104 | rs492338 | rs762173 | rs9565661 | rs2027605 |
| rs12457191 | rs2000416 | rs2835121 | rs492346 | rs762227 | rs9565968 | rs2836338 |
| rs12458066 | rs2000490 | rs2835169 | rs492597 | rs7624098 | rs9566836 | rs2836358 |
| rs12458637 | rs2000833 | rs2835293 | rs4927236 | rs7624366 | rs9567448 | rs4941183 |
| rs12458713 | rs2004000 | rs2835349 | rs492781 | rs762438 | rs9567700 | rs4941388 |
| rs12482086 | rs2005187 | rs2835567 | rs4939701 | rs7626725 | rs9568497 | rs7728402 |
| rs12482146 | rs2006089 | rs2835695 | rs4939702 | rs7633784 | rs9568684 | rs7733022 |
| rs12482714 | rs200680 | rs2835704 | rs4939735 | rs7634577 | rs9568713 | rs9573927 |
| rs12482786 | rs2009879 | rs2835722 | rs4940009 | rs7639145 | rs9569550 | rs9574740 |
| rs12483578 | rs2012898 | rs2835723 | rs4940235 | rs7639867 | rs9570226 | |
| rs12490235 | rs2012982 | rs2835735 | rs4940498 | rs7651989 | rs9570290 | |
| rs12513430 | rs2013669 | rs2835790 | rs4940563 | rs765557 | rs9570447 | |
| rs12514412 | rs2014509 | rs2835802 | rs4940615 | rs7661729 | rs9571811 | |
| rs1253809 | rs2014678 | rs2835823 | rs4940791 | rs7702862 | rs9571821 | |
| rs1253811 | rs2018093 | rs2835955 | rs4940955 | rs7711972 | rs9572020 | |
| rs12561781 | rs2019006 | rs2835965 | rs4940957 | rs7716283 | rs9572196 | |
| rs12565445 | rs2025951 | rs2835971 | rs4940960 | rs7717101 | rs9572308 | |
| rs125810 | rs2026263 | rs2835975 | rs4941085 | rs7721965 | rs9573824 | |

**Table 7: Markers used in the present invention**

| Marker number | Marker number | Marker number | Marker number | Marker number | Marker number | Marker number |
|---|---|---|---|---|---|---|
| rs12585235 | rs2031546 | rs2836656 | rs4941939 | rs7807853 | rs9574898 | rs12960453 |
| rs12586094 | rs2032313 | rs2836661 | rs4942060 | rs7822979 | rs9574900 | rs12961253 |
| rs12604515 | rs203332 | rs2836706 | rs4942169 | rs7831906 | rs9575364 | rs2104632 |
| rs12604519 | rs2037920 | rs2836837 | rs4942242 | rs7856187 | rs9575369 | rs2111299 |
| rs12605543 | rs2037921 | rs2836840 | rs4942416 | rs786018 | rs9575372 | rs2837751 |
| rs12605917 | rs2039056 | rs2836842 | rs4942486 | rs7914609 | rs9579214 | rs2837801 |
| rs12605932 | rs2039281 | rs2836943 | rs4942642 | rs794185 | rs9581121 | rs525776 |
| rs12606001 | rs2039622 | rs2836956 | rs4942769 | rs7967526 | rs9583190 | rs526057 |
| rs12626853 | rs2043428 | rs2836958 | rs4942830 | rs797517 | rs9583537 | rs7997078 |
| rs12626876 | rs2044800 | rs2836975 | rs4942931 | rs7981995 | rs9583996 | rs7997881 |
| rs12627315 | rs2046845 | rs2836980 | rs4943119 | rs7982563 | rs958687 | rs977660 |
| rs12627610 | rs2051121 | rs2836985 | rs4943694 | rs7982833 | rs9592665 | rs9783885 |
| rs12627745 | rs2051189 | rs2837129 | rs4943696 | rs7983168 | rs9593922 | rs12959212 |
| rs12630707 | rs2051382 | rs2837302 | rs4949256 | rs7983218 | rs9597134 | rs12960451 |
| rs12637291 | rs2057529 | rs2837381 | rs495737 | rs7984225 | rs9600079 | rs2099255 |
| rs12659620 | rs2058276 | rs2837393 | rs496627 | rs7984261 | rs9601268 | rs2100750 |
| rs12724092 | rs2059757 | rs2837395 | rs4986223 | rs7984523 | rs9601567 | rs2837738 |
| rs1274749 | rs2060816 | rs2837399 | rs4989135 | rs7984835 | rs9604328 | rs2837747 |
| rs12756081 | rs2063222 | rs2837403 | rs499416 | rs7986681 | rs9617452 | rs522505 |
| rs1276034 | rs2065280 | rs2837411 | rs4998815 | rs7988095 | rs962267 | rs524566 |
| rs12763013 | rs2065288 | rs2837490 | rs500910 | rs7988209 | rs9634593 | rs7995700 |
| rs128365 | rs2067741 | rs2837494 | rs501062 | rs7989235 | rs9636883 | rs7996275 |
| rs1284419 | rs2068051 | rs2837512 | rs5023173 | rs798963 | rs9636977 | rs970705 |
| rs12858753 | rs2070535 | rs2837529 | rs508151 | rs7989798 | rs9637300 | rs975336 |
| rs12859190 | rs2071754 | rs2837553 | rs509215 | rs7990298 | rs9646522 | |
| rs12864209 | rs2073425 | rs2837592 | rs509741 | rs7992072 | rs9646629 | |
| rs12876644 | rs2076237 | rs2837637 | rs512699 | rs7992416 | rs9647139 | |
| rs12925084 | rs208932 | rs2837655 | rs513775 | rs7993087 | rs9647235 | |
| rs12936110 | rs2090036 | rs2837701 | rs514556 | rs7993804 | rs9647276 | |
| rs12953319 | rs2094186 | rs2837705 | rs514669 | rs7994585 | rs9652107 | |
| rs12955787 | rs2096507 | rs2837712 | rs515391 | rs7994654 | rs9675925 | |
| rs12957246 | rs2096905 | rs2837717 | rs515551 | rs7995283 | rs9676063 | |
| rs12957256 | rs2097096 | rs2837736 | rs515920 | rs7995306 | rs968906 | |

**Table 8: Markers used in the present invention**

| Marker number | Marker number | Marker number | Marker number | Marker number | Marker number | Marker number |
|---|---|---|---|---|---|---|
| rs12961631 | rs2113462 | rs2837865 | rs532095 | rs7997893 | rs9785659 | rs1326056 |
| rs12961741 | rs2115980 | rs2838004 | rs532625 | rs7997966 | rs9785716 | rs13275667 |
| rs12961750 | rs2116378 | rs2838081 | rs535923 | rs7998641 | rs9785897 | rs2187091 |
| rs12962651 | rs211962 | rs2838104 | rs536419 | rs7999126 | rs9786101 | rs2188584 |
| rs12963212 | rs2120204 | rs2838125 | rs537435 | rs7999812 | rs9786111 | rs2849977 |
| rs12963466 | rs212315 | rs2838304 | rs545723 | rs8000390 | rs9786121 | rs2850125 |
| rs12965753 | rs2136681 | rs2838361 | rs550801 | rs8001960 | rs9786140 | rs608382 |
| rs12966281 | rs2137492 | rs2838438 | rs556046 | rs8002541 | rs9786194 | rs608713 |
| rs12966492 | rs214054 | rs2838441 | rs558700 | rs803815 | rs9786276 | rs8091446 |
| rs12967515 | rs214341 | rs2838568 | rs559372 | rs8083067 | rs9786291 | rs8091825 |
| rs12967616 | rs2146442 | rs2838724 | rs560169 | rs8083437 | rs9786386 | rs9909561 |
| rs12968141 | rs2148443 | rs2838799 | rs561418 | rs8083682 | rs9786773 | rs991045 |
| rs12968648 | rs2149436 | rs2838806 | rs569216 | rs8084206 | rs9786824 | rs1325798 |
| rs12969413 | rs2150419 | rs2838813 | rs575936 | rs8084711 | rs9786876 | rs1325968 |
| rs12969725 | rs2151277 | rs2838815 | rs5761308 | rs8084792 | rs9786885 | rs2183557 |
| rs12971228 | rs2154487 | rs2838820 | rs5764891 | rs8085054 | rs9788296 | rs2186557 |
| rs13046156 | rs2154549 | rs2838887 | rs576808 | rs8085056 | rs9789153 | rs2849697 |
| rs13046342 | rs2154550 | rs2838890 | rs578835 | rs8085222 | rs9805596 | rs2849865 |
| rs1304747 | rs2154723 | rs2838893 | rs581394 | rs8086286 | rs9805694 | rs607127 |
| rs13049234 | rs2155797 | rs2839287 | rs582547 | rs8086449 | rs9805804 | rs6072085 |
| rs13049853 | rs2156187 | rs2839377 | rs582853 | rs8086752 | rs9813365 | rs8091123 |
| rs13050660 | rs2156384 | rs2839386 | rs585632 | rs8086807 | rs9818400 | rs8091380 |
| rs13052088 | rs2156650 | rs2839392 | rs591173 | rs8087052 | rs982328 | rs9891988 |
| rs13087163 | rs2160043 | rs2839468 | rs591498 | rs8087127 | rs9828270 | rs990557 |
| rs13152923 | rs2161775 | rs2839470 | rs593340 | rs8087403 | rs9835007 | |
| rs13159598 | rs2166029 | rs2839508 | rs595106 | rs8087551 | rs9837159 | |
| rs13162651 | rs2174524 | rs2839520 | rs596778 | rs8087849 | rs9845467 | |
| rs13163878 | rs2174571 | rs2842906 | rs599551 | rs8088596 | rs984659 | |
| rs13168731 | rs2174896 | rs28468602 | rs599881 | rs8088779 | rs985035 | |
| rs13178296 | rs2178841 | rs2848957 | rs6014601 | rs8088832 | rs985198 | |
| rs13200025 | rs2178848 | rs2848958 | rs602212 | rs8089359 | rs9853755 | |
| rs1323556 | rs2181753 | rs2848961 | rs6047745 | rs8089613 | rs9861671 | |
| rs1325453 | rs2182957 | rs2849253 | rs607020 | rs8090831 | rs9869577 | |

**Table 9: Markers used in the present invention**

| Marker number | Marker number | Marker number | Marker number | Marker number | Marker number | Marker number |
|---|---|---|---|---|---|---|
| rs1328368 | rs2198683 | rs2850542 | rs6108022 | rs8092218 | rs993930 | rs1378492 |
| rs1328926 | rs220128 | rs2852146 | rs612573 | rs8092926 | rs9944568 | rs1378800 |
| rs13304168 | rs220149 | rs2861624 | rs6137476 | rs8094161 | rs9945284 | rs2246422 |
| rs13304202 | rs220171 | rs28649411 | rs614290 | rs8094280 | rs9945648 | rs2247021 |
| rs1331948 | rs220268 | rs287355 | rs616669 | rs8095071 | rs9945969 | rs3121808 |
| rs1331951 | rs2203754 | rs2873580 | rs619542 | rs8095250 | rs9947210 | rs3127637 |
| rs1333023 | rs2205533 | rs2878293 | rs625028 | rs8095514 | rs9947426 | rs6492589 |
| rs1333027 | rs2206747 | rs2878901 | rs625090 | rs8095747 | rs9947829 | rs6506138 |
| rs1333072 | rs2211681 | rs2885243 | rs626519 | rs8096263 | rs9948368 | rs8131559 |
| rs1334384 | rs2211845 | rs2887596 | rs627527 | rs8096542 | rs9948679 | rs8132424 |
| rs1335282 | rs2211869 | rs2892463 | rs628221 | rs8096605 | rs9948733 | rs9959180 |
| rs1335787 | rs2211938 | rs2897977 | rs630706 | rs8096830 | rs9948841 | rs9959555 |
| rs1335788 | rs2211973 | rs2901821 | rs6311 | rs8097023 | rs9948974 | rs1370079 |
| rs13380936 | rs2212624 | rs2921452 | rs632324 | rs8097306 | rs9949020 | rs1377341 |
| rs13381153 | rs2212626 | rs293105 | rs632678 | rs8097433 | rs9949323 | rs2245411 |
| rs13381188 | rs2212809 | rs2933307 | rs632683 | rs8097467 | rs9949565 | rs2246122 |
| rs1340312 | rs2212828 | rs2941782 | rs632986 | rs8097792 | rs9949574 | rs3106603 |
| rs1340333 | rs2217442 | rs2946523 | rs634293 | rs8097822 | rs9949868 | rs3118045 |
| rs1340562 | rs2222370 | rs2953258 | rs634760 | rs8098182 | rs9949882 | rs6492379 |
| rs13433508 | rs2222999 | rs2953261 | rs639862 | rs8098925 | rs9950906 | rs6492586 |
| rs1345492 | rs2223079 | rs2969931 | rs640254 | rs8099616 | rs9951809 | rs8130781 |
| rs1348466 | rs2226356 | rs2993502 | rs641366 | rs8099832 | rs9951893 | rs8131481 |
| rs1349094 | rs2226358 | rs2997116 | rs6426721 | rs8127266 | rs9952107 | rs9958812 |
| rs1349936 | rs2226798 | rs3011522 | rs6439686 | rs8127332 | rs9952148 | rs9958938 |
| rs13503 | rs2226859 | rs3014944 | rs6442180 | rs8127569 | rs9952357 | |
| rs1351407 | rs2236483 | rs3015419 | rs645539 | rs8127634 | rs9952908 | |
| rs13554 | rs2236944 | rs3019879 | rs645699 | rs8128478 | rs9953136 | |
| rs1358368 | rs2241585 | rs304838 | rs6469456 | rs8128523 | rs9954012 | |
| rs1361029 | rs2242661 | rs306395 | rs6483561 | rs8128650 | rs9954208 | |
| rs1361768 | rs2242752 | rs3091601 | rs6490683 | rs8129332 | rs9954439 | |
| rs1364416 | rs2242753 | rs3096835 | rs6490713 | rs8129919 | rs9955860 | |
| rs1365251 | rs2243936 | rs3101866 | rs6490946 | rs8130292 | rs9957425 | |
| rs1369348 | rs2244188 | rs3106556 | rs6491350 | rs8130587 | rs9957591 | |

**Table 10: Markers used in the present invention**

| Marker number | Marker number | Marker number | Marker number | Marker number | Marker number | Marker number |
|---|---|---|---|---|---|---|
| rs1379823 | rs2247221 | rs3171532 | rs6506332 | rs8132865 | rs9959597 | rs1472406 |
| rs1380332 | rs2248218 | rs326046 | rs6506837 | rs8132870 | rs9959723 | rs1473279 |
| rs1382394 | rs2249360 | rs328125 | rs6507196 | rs8133195 | rs9960454 | rs1474092 |
| rs1385338 | rs2250226 | rs329027 | rs6507440 | rs8134612 | rs9961499 | rs1478526 |
| rs1389561 | rs2250494 | rs331018 | rs6507719 | rs8134986 | rs9963406 | rs2284636 |
| rs1390431 | rs2250926 | rs331020 | rs6507783 | rs8181861 | rs9963983 | rs2287434 |
| rs1412819 | rs2251085 | rs334458 | rs6507967 | rs8184900 | rs9964749 | rs2289152 |
| rs1412822 | rs2251210 | rs336214 | rs6508168 | rs825977 | rs9964911 | rs229441 |
| rs1413021 | rs2252046 | rs336279 | rs6508266 | rs844974 | rs9964940 | rs375484 |
| rs1413158 | rs2252776 | rs340966 | rs6508351 | rs844975 | rs9965174 | rs375886 |
| rs1413435 | rs2252828 | rs341237 | rs6508502 | rs844978 | rs9965410 | rs3760582 |
| rs1415019 | rs225383 | rs341499 | rs651029 | rs844986 | rs9965900 | rs377191 |
| rs1417313 | rs225396 | rs341506 | rs651407 | rs844990 | rs9966050 | rs6562599 |
| rs1417907 | rs2254368 | rs347128 | rs6516794 | rs844999 | rs9966798 | rs6562888 |
| rs1421182 | rs2255059 | rs349714 | rs6516819 | rs845015 | rs9967142 | rs6563329 |
| rs1424406 | rs2255332 | rs352247 | rs6517605 | rs845017 | rs9967277 | rs6565830 |
| rs1430378 | rs2256000 | rs35379414 | rs6518100 | rs845018 | rs9967440 | rs892430 |
| rs1430381 | rs2256417 | rs355708 | rs6518252 | rs845022 | rs9967534 | rs894050 |
| rs1437650 | rs2269145 | rs367841 | rs652539 | rs845024 | rs996906 | rs896036 |
| rs1442134 | rs2269161 | rs369906 | rs6550169 | rs845969 | rs9974136 | rs898484 |
| rs1445728 | rs2269173 | rs372883 | rs6550215 | rs857569 | rs997416 | rs9976426 |
| rs1446770 | rs2274328 | rs373037 | rs655209 | rs858044 | rs9974225 | rs9977055 |
| rs1447740 | rs2274403 | rs3736867 | rs6555064 | rs863075 | rs9974317 | rs9977610 |
| rs1451940 | rs2274463 | rs3736972 | rs6561105 | rs864674 | rs9974879 | rs9977815 |
| rs1452670 | rs2274774 | rs3737893 | rs6561326 | rs875625 | rs9974970 | |
| rs1455514 | rs2276218 | rs3742188 | rs6561605 | rs876165 | rs9975304 | |
| rs1455872 | rs2277798 | rs3744877 | rs6561644 | rs877786 | rs9975452 | |
| rs1464236 | rs2279962 | rs3744998 | rs6561709 | rs877856 | rs9975831 | |
| rs1465509 | rs2281767 | rs3746897 | rs6561727 | rs878971 | rs997587 | |
| rs1467756 | rs2284214 | rs3746924 | rs6561900 | rs883868 | rs9976123 | |
| rs1471171 | rs2284514 | rs3751405 | rs6561924 | rs888789 | rs9976168 | |

In the present invention, the biological sample may be blood, plasma, serum, urine, or saliva.

In the present invention, the marker sequences may have genotypes as shown in Table 11 below for each SNP site, and thus any SNP combination (red) cannot provide information useful for prediction of organ transplant rejection, and any SNP combinations (yellow and green) can provide useful information which is entirely determined according to a random distribution of donor-specific and recipient-specific SNP genes.

**Table 11: Donor/recipient SNP combinations predicted by analysis using selected marker set**

| Donor/recipient | X:X | X:Y | Y:Y | | |
|---|---|---|---|---|---|
| X:X | N | MI | HI | HI | Highly Informative |
| X:Y | HI | NI | HI | MI | Moderately Informative |
| Y:Y | HI | MI | NI | NI | No Informative |

For example, when an SNP marker combination having a minor allele frequency of 45% is used in the analysis, the ratio between each of donor-specific alleles and each of recipient-specific alleles, calculated by the Hardy-Weinberg equilibrium, is represented in Table 12 below.

**Table 12: Allele ratios predicted by analysis using an SNP marker combination having a minor allele frequency of 45%**

| X=55% Y=45% | X:X (30%) | X:Y (49%) | Y:Y (21%) | | |
|---|---|---|---|---|---|
| X:X (30%) | 9.0% | 14.7% | 6.3% | HIGH | 37.6% |
| X:Y (49%) | 14.7% | 24.0% | 10.3% | MEDIUM | 25.0% |
| Y:Y (21%) | 6.3% | 10.3% | 4.4% | LOW | 37.4% |

In the present invention, the step of amplifying the marker sequences may further comprise amplifying all of the markers shown in Tables 1 to 10.

In the present invention, the ratio between the marker sequences might imply the ratio between the amount of each donor-derived marker sequence and the amount of each recipient-derived marker sequence, selected from the list of markers shown in Tables 1 to 10.

The NGS platform that is used in the present invention is optimized for analysis of sequence fragments having a size of 100 bp. Essential factors to be taken into consideration while making a choice of the NGS platform includes the read-lenth that is readable at the same time, basic error rate, analysis speed, and reaction efficiency.

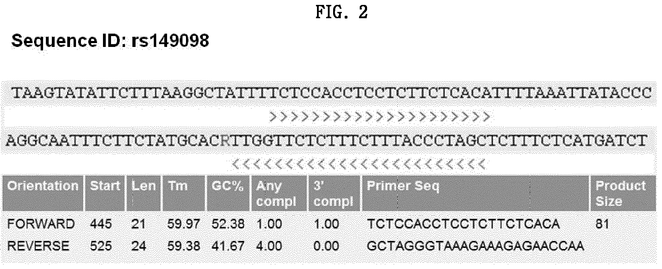

In the present invention, it was shown that when the markers listed in Tables 1 to 10 were amplified in order to optimize the above-described factors, a desired SNP site was within 35 bp from the starting point of sequencing, and the average length of amplified marker sequences were 70 bp (FIG. 2) .

Therefore, in the present invention, the amplified marker sequences in the biological sample may be less than 200 bp in length.

The markers that are used in the present invention are bi-allelic SNP sites which are the markers whose positions and expected nucleotide sequences are all known. Thus, when the nucleotide at any position differs from a known nucleotide (for example, A is read in place of the correct nucleotide G/T), it can be counted as an error.

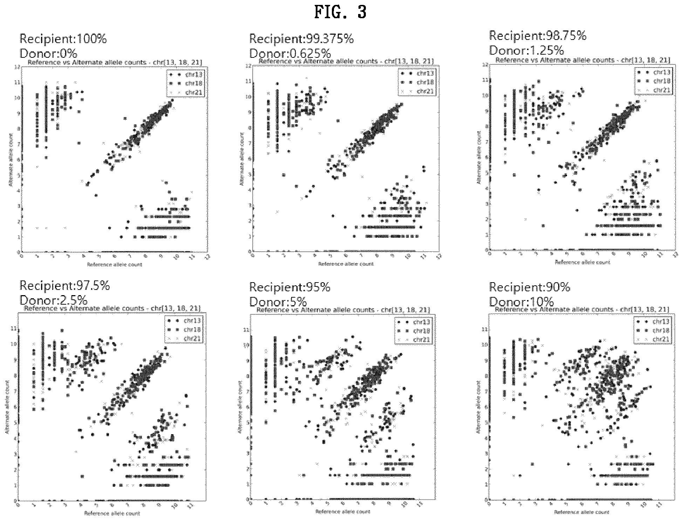

By analyzing 2023 markers as represented in FIG. 3, it was inferred that an error rate could be easily calculated.

In the present invention, the ratio between the marker sequences may be calculated along with a sequencing error rate.

In the present invention, the cutoff values may be reference values established from a normal biological sample.

Meanwhile, it was found that organ transplant rejection can be predicted by observing a time-dependent change in the amount of donor-derived DNA in a recipient who received an organ.

In another example of the present invention, biological samples were obtained from a recipient, who received an organ, before and immediately after organ transplantation, and then were obtained at certain time intervals after organ transplantation.

The obtained biological samples were analyzed, and as a result, it was observed that the ratio of donor-derived SNP markers were increased when organ transplant rejection occurred.

Therefore, in another aspect, the present invention is directed to a method of predicting organ transplant rejection in a biological sample, obtained from a recipient who received an organ from a donor, by next-generation sequencing (NGS) or digital base amplification, the method comprising the steps of:
non-invasively obtaining a biological sample, which contains donor-derived and recipient-derived cell-free nucleic acid molecules, from a recipient who received an organ from a donor;
amplifying three or more marker sequences, selected from marker sequences shown in Tables 1 to 10, in cell-free nucleic acid molecules isolated from the biological sample;
analyzing the amplified sequences by next-generation sequencing (NGS) or digital base amplification;
based on the analysis of the sequences, determining the ratio between each of the donor-derived marker sequences and each of the recipient-derived marker sequences; and
measuring the ratio over time, and predicting whether the recipient will have transplant rejection, graft dysfunction or organ failure when the ratio each of the donor-derived marker sequences increases.

In the present invention, the biological sample may be blood, plasma, serum, urine, or saliva.

In the present invention, the step of amplifying the marker sequences further comprises amplifying all of the markers listed in Tables 1 to 10.

In the present invention, the ratio between the marker sequences might imply the ratio between the amount of each donor-derived marker sequence and the amount of each recipient-derived marker sequence, selected from the markers shown in Tables 1 to 10.

In the present invention, the ratio between the marker sequences may be calculated along with a sequencing error rate.

In the present invention, the amplified marker sequences in the biological sample might be less than 200 bp in length.

In the present invention, the ratio measurement time may be selected from the group consisting of before organ transplantation, immediately after organ transplantation, and one day, two days, one week, one month, two months, three months, one year, two years, and 10 years after organ transplantation.

The present invention is also directed to a computer system comprising a computer readable medium encoded with a plurality of instructions for controlling a computing system to perform an operation of predicting organ transplant rejection in a biological sample, obtained from a recipient who received an organ from a donor, by use of next-generation sequencing (NGS) or digital base amplification,
wherein the biological sample contains donor-derived and recipient-derived cell-free nucleic acid molecules from a recipient who received an organ from a donor, and
the operation comprises the steps of:
receiving data obtained by analyzing three or more marker sequences, selected from markers shown in Tables 1 to 10, in the cell-free nucleic acid molecules isolated from the biological sample, by use of next-generation sequencing (NGS) or digital base amplification;
based on the analysis of the sequences, determining the ratio between each of the donor-derived marker sequences and each of the recipient-derived marker sequences;
comparing the ratio with one or more cutoff values; and
based on the comparison, predicting whether or not organ transplant rejection in the recipient will be present.

### EXAMPLES

Hereinafter, the present invention will be described in further detail with reference to examples. It will be obvious to a person having ordinary skill in the art that these examples are for illustrative purposes only and are not to be construed to limit the scope of the present invention. Thus, the substantial scope of the present invention will be defined by the appended claims and equivalents thereof.

### Example 1: Prediction of Organ Transplant Rejection in Artificially Generated Organ Transplant Recipients

### 1.1: Pretreatment for Preparation and Analysis of Artificial DNA Samples from Organ Transplant Recipients

Male DNA (donor) was mixed with female DNA (recipient) such that the percentage of the male DNA in the female DNA would be 0%, 0.625%, 1.25%, 2.5%, 5% or 10%, thereby preparing artificial organ transplant patient genomic DNA samples.

To perform a TruSeq Custom Amplicon (TSCA) assay (Illumina, USA) using 100 ng of each gDNA, Custom Amplicon was prepared. A heat block was adjusted to 95°C, and 5 µl of each of DNA and CAT (Custom Amplicon Oligo Tube) was added to a 1.7-ml tube. As control reagents, 5 µl of each of ACD1 and ACP1 was also prepared. 40 µl of OHS1 (Oligo Hybridization for Sequencing Reagent 1) was added to each tube and mixed well using a pipette, and each tube was maintained at 95°C for 1 min, and subjected to oligo hybridization at 40°C for 80 min subsequently. following this, the temperature was lowered and 45 µl of SW1 (stringent wash 1) reagent was added to an FPU (Filter Plate Unit) plate membrane, followed by centrifugation at 2,400 x g and 20°C for 10 min. The sample tube was subjected to hybridization, spun-down, and the sample was transferred to the FPU plate using a pipette, and then centrifuged at 2,400 x g and 20°C for 2 min. This was followed by the washing of the sample twice with 45 µl of SW1 reagent, and addition of 45 µl of UB1 (Universal Buffer 1) reagent. Subsequently, centrifugation was performed under the same conditions to remove unreacted unbound oligo.For extension-ligation of hybridized oligo, 45 µl of ELM3 (extension-ligation mix 3) was added to the sample covered with a foil and incubated in an incubator at 37°C for 45 min. After completion of the incubation, the foil was removed, and the sample was centrifuged at 2,400 x g for 2 min. Then, 25 µl of 50 mM NaOH was added to the sample which was then pipetted 5 to 6 times using a pipette and incubated at room temperature for 5 minutes. During the incubation, a PMM2/TDP1 (PCR Master Mix 2 / TruSeq DNA Polymerase 1) mixture was added to the PCR tube containing P5 and P7 index. After completion of the incubation, 20 µl of DNA diluted in NaOH was added, thereby preparing a total of 50 µl of a PCR amplification sample. The prepared sample was subjected to PCR reaction under the following conditions:
<PCR conditions>
   -95°C, 3min
   -28 cycles
   95°C, 30sec
   66°C, 30sec
   72°C, 60sec
   -72°C, 5min
   -Hold at 10°C

After completion of the PCR cycles, the sample was analyzed using a QIAxcel system for confirmation. The sample was then purified using 60 µl of beads and suspended in 30 µl of resuspension buffer (RS).

### 1.2: Analysis of Artificial DNA Sample from Organ Transplant Recipient

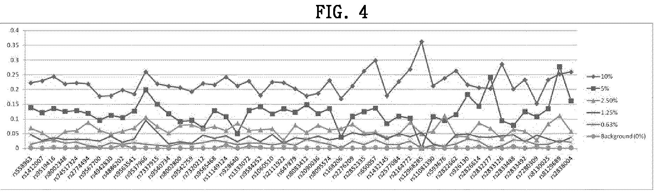

It was observed that the sample could be sequenced using a sequencing system and the corresponding markers could be counted, making it possible to monitor organ transplant rejection through an algorithm and a pipeline (FIGS. 3 and 4).

Allele counts corresponding to the SNP markers identified using next-generation sequencing were graphically shown. On the X-axis, reference allele or major allele counts were expressed, and on the Y-axis, alternate allele or minor allele counts were expressed as log2 values (FIG. 3). Particularly, because the selected SNP markers were SNPs located at chromosome 13, 18 and 21, these markers were indicated by blue (●), green (■) and red (×), respectively (FIG. 3).

As represented in Table 13 below, the mixed DNAs may show a total of 9 phenotypes.

**Table 13: Phenotypes of mixed (organ transplant patient) DNAs**

| M F | **AA** | **Aa** | **aa** |
|---|---|---|---|
| **AA** | AAAA | AAAa | AAaa |
| **Aa** | AaAA | AaAa | Aaaa |
| **aa** | aaAA | aaAa | aaaa |

The phenotypes of the artificially prepared DNA may appear as AA, Aa, aA and aa, and thus have the possibility of eight distributions (AAaa and aaAA are regarded as the same phenotype). It could be seen that, as the donor-derived DNA increased, the AaAA and Aaaa distributions increased at a constant rate (FIG. 3).

As represented in FIG. 3, the distribution of the donor-derived biomarkers changes depending on the degree of mixing of the biomarkers. When this distribution is quantitatively measured and calculated, small amounts of the donor-derived genes present in the recipient's blood can be detected or measured, and when the amount of the donor-derived gene mutation is measured and observed, organ transplant rejection in the recipient can be predicted or observed.

In addition, as represented in Table 14 below, when two DNAs were mixed in different amounts and were actually measured by next-generation sequencing, the amounts of the mixed DNAs could be accurately measured even when present in minute amounts by quantitatively analyzing biomarkers using next-generation sequencing.

**Table 14: Experimental Ratio and SNP Counting-Based Ratio of Mixed DNAs**

| | Background (0% ) | 10% | 5% | 2.50% | 1.25% | 0.63% |
|---|---|---|---|---|---|---|
| Fractio n Homo | 0.001395983 | 0.22144 3 | 0.12378 6 | 0.06368 8 | 0.03500 9 | 0.01580 2 |
| Fractio n Hetero | 0.001495645 | 0.11011 2 | 0.06094 4 | 0.03242 5 | 0.01755 1 | 0.01092 1 |
| Actual Fractio n | | 22.08% | 12.28% | 6.43% | 3.51% | 1.88% |

In this context, when bases corresponding to AA and aa of artificially mixed donor-derived DNA are counted, values as listed in Table 14 above can be obtained. Although there is a difference of about 2 folds between the experimental value of mixed DNA and the value measured by analysis, this difference may have appeared because the measured DNA amount is not an absolute amount.

As shown in FIG. 4, although markers differ from each other, the use of several markers makes it possible to accurately measure or observe organ transplant rejection.

When the method of the present invention is actually applied to patients, donor-derived DNA can be expressed as a numerical value at varying time points, and organ transplant rejection can be monitored.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

### INDUSTRIAL APPLICABILITY

The method of the present invention is useful for non-invasive prediction and monitoring of organ transplant rejection, and thus it has a high industrial applicability.

## Claims

1. A method of predicting organ transplant rejection in a biological sample, obtained from a recipient who received an organ from a donor, by next-generation sequencing (NGS) or digital base amplification, the method comprising the steps of:
obtaining a biological sample non-invasively, which contains donor-derived and recipient-derived cell-free nucleic acid molecules, from a recipient who received an organ from a donor;
amplifying three or more marker sequences, selected from marker sequences shown in Tables 1 to 10, in cell-free nucleic acid molecules isolated from the biological sample;
analyzing the amplified sequences by next-generation sequencing (NGS) or digital base amplification;
based on the analysis of the sequences, determining the ratio between each of the donor-derived marker sequences and each of the recipient-derived marker sequences; and
comparing the ratio with one or more cutoff values.

2. The method of claim 1, wherein the biological sample is blood, plasma, serum, urine, or saliva.

3. The method of claim 1, wherein the step of amplifying the marker sequences further comprises amplifying all of the markers listed in Tables 1 to 10.

4. The method of claim 1, wherein the ratio between the marker sequences is the ratio between the amount of each donor-derived marker sequence and the amount of each recipient-derived marker sequence, selected from the markers shown in Tables 1 to 10.

5. The method of claim 1, wherein the ratio between the marker sequences is be calculated along with a sequencing error rate.

6. The method of claim 1, wherein the amplified marker sequences in the biological sample are less than 200 bp in length.

7. The method of claim 1, wherein the cutoff values are reference values established from a normal biological sample.

8. A method of predicting organ transplant rejection in a biological sample, obtained from a recipient who received an organ from a donor, by next-generation sequencing (NGS) or digital base amplification, the method comprising the steps of:
obtaining a biological sample non-invasively, which contains donor-derived and recipient-derived cell-free nucleic acid molecules, from a recipient who received an organ from a donor;
amplifying three or more marker sequences, selected from marker sequences shown in Tables 1 to 10, in cell-free nucleic acid molecules isolated from the biological sample;
analyzing the amplified sequences by next-generation sequencing (NGS) or digital base amplification;
based on the analysis of the sequences, determining the ratio between each of the donor-derived marker sequences and each of the recipient-derived marker sequences; and
measuring the ratio over time, and predicting whether the recipient will have transplant rejection, graft dysfunction or organ failure when the ratio each of the donor-derived marker sequences increases.

9. The method of claim 8, wherein the biological sample is blood, plasma, serum, urine, or saliva.

10. The method of claim 8, wherein the step of amplifying the marker sequences further comprises amplifying all of the markers shown in Tables 1 to 10.

11. The method of claim 8, wherein the ratio between the marker sequences is the ratio between the amount of each donor-derived marker sequence and the amount of each recipient-derived marker sequence, selected from the markers shown in Tables 1 to 10.

12. The method of claim 8, wherein the ratio between the marker sequences is calculated along with a sequencing error rate.

13. The method of claim 8, wherein the amplified marker sequences in the biological sample are less than 200 bp in length.

14. The method of claim 8, wherein the ratio measurement time is selected from the group consisting of before organ transplantation, immediately after organ transplantation, and one day, two days, one week, one month, two months, three months, one year, two years, and 10 years after organ transplantation.

15. A computer system comprising a computer readable medium encoded with a plurality of instructions for controlling a computing system to perform an operation of predicting organ transplant rejection in a biological sample, obtained from a recipient who received an organ from a donor, by use of next-generation sequencing (NGS) or digital base amplification,
wherein the biological sample contains donor-derived and recipient-derived cell-free nucleic acid molecules from a recipient who received an organ from a donor, and
wherein the operation comprises the steps of:
receiving data obtained by analyzing three or more marker sequences, selected from markers shown in Tables 1 to 10, in the cell-free nucleic acid molecules isolated from the biological sample, by use of next-generation sequencing (NGS) or digital base amplification;
based on the analysis of the sequences, determining the ratio between each of the donor-derived marker sequences and each of the recipient-derived marker sequences;
comparing the ratio with one or more cutoff values; and
based on the comparison, predicting whether or not organ transplant rejection in the recipient will be present.
